# EUROPÄISCHE PATENTSCHRIFT

(11) **EP 1 677 111 B1**
(45) Veröffentlichungstag und Bekanntmachung des Hinweises auf die Patenterteilung: **05.12.2007**
(21) Anmeldenummer: 04030547.6
(22) Anmeldetag: 23.12.2004
(51) Int. Cl.: G01N 33/543

(54) **Verfahren zum Herstellen eines Sensorchips und Sensorchip-Rohling**
Method for producing a biochip and blank biochip
Méthode de fabrication d'une biopuce et biopuce vierge

(43) Veröffentlichungstag der Anmeldung: 05.07.2006
(73) Patentinhaber: Micronas Holding GmbH, 79108 Freiburg (DE)
(72) Erfinder: Klapproth, Holger, Dr., 79108 Freiburg (DE)
(74) Vertreter: Huwer, Andreas

(56) Entgegenhaltungen:
- WO-A-03/005013
- WO-A-20/04050830
- WO-A2-20/04005477
- DE-C1- 4 436 173
- ZHOU YE ET AL: "Structural characterization of microcontact printed arrays of hexa(ethylene glycol)-terminated alkanethiols on gold." LANGMUIR : THE ACS JOURNAL OF SURFACES AND COLLOIDS. 20 JUL 2004, Bd. 20, Nr. 15, 20. Juli 2004 (2004-07-20), Seiten 6206-6215, XP002347785 ISSN: 0743-7463
- YADAVALLI V.K. ET AL: 'Microfabricated protein-containing poly(ethylene glycol) hydrogel arrays for biosensing' SENSORS AND ACTUATORS B Bd. 97, Nr. 2-3, 01 Februar 2004, ELSEVIER SEQUOIA S.A., LAUSANNE, CH, Seiten 290 - 297, XP004486630

## Beschreibung

Die Erfindung betrifft ein Verfahren nach dem Oberbegriff von Anspruch 1 und einen Sensorchip-Rohling nach dem Oberbegriff von Anspruch 5.

Ein Verfahren zum Herstellen eines nach dem Rezeptor-Liganden-Prinzip arbeitenden Sensorchips ist aus DE 102 33 303 A1 bekannt. Dabei werden auf einem Oberflächenbereich eines Trägers Rezeptoren kovalent immobilisiert. Die Rezeptoren werden zunächst in einer Pufferlösung gelöst und danach wird die Pufferlösung auf den Oberflächenbereich aufgebracht. Bei einer Pufferlösung ändert sich der pH-Wert bei Zugabe einer Säure oder Base wesentlich weniger stark, als dies bei einer ungepufferten Lösung der Fall ist. Das Aufbringen der Pufferlösung mit den darin enthaltenen Rezeptoren erfolgt bevorzugt mit Hilfe eines Druckers, beispielsweise eines Nadeldruckers und/oder eines Piezostackdruckers.

Ein Rezeptor kann Nukleinsäuren oder Derivate davon (DNA, RNA, PNA, LNA, Oligonukleotide, Plasmide, Chromosomen), Peptide, Proteine (Enzym, Protein, Oligopeptide, zelluläre Rezeptorproteine und deren Komplexe, Peptidhormone, Antikörper und deren Fragmente), Kohlenhydrate und deren Derivate, insbesondere glykosylierte Proteine und Glycoside, Fette, Fettsäuren und/oder Lipide umfassen.

Die kovalente Bindung des Rezeptors an den Oberflächenbereich ist vom pH-Wert und der Pufferkapazität der Pufferlösung abhängig. Allerdings lassen sich nicht alle gewünschten Pufferlösungen zuverlässig drucken. So sind insbesondere Pufferlösungen, die aufgrund ihres pH-Wertes eine relativ hohe Salzkonzentration aufweisen, problematisch, weil sie dazu neigen, sehr schnell auszukristallisieren. Durch die Kristalle kann die Kapillare oder Düse der Drucknadel verstopfen. Auch kann es passieren, dass die Pufferlösung bereits kristallisiert, bevor sie zum Drucker gelangt, beispielsweise in einer Aufnahmevertiefung einer Mikrotiterplatte, in der die Pufferlösung bevorratet wird. Ungünstig ist außerdem, dass empfindliche Rezeptoren oft gekühlt werden müssen, damit sie haltbar bleiben. Durch die Kühlung wird aber die Gefahr der Kristallisierung noch vergrößert.

Um eine effektive kovalente Bindung der Rezeptoren an den Träger zu erreichen, ist ein bestimmter pH-Wert erforderlich, der durch die Pufferkapazität gewährleistet werden soll. Bei hohen Rezeptor-Konzentrationen braucht man folglich auch hohe Salzkonzentrationen in der Pufferlösung. Diese lassen sich in der Praxis aber nicht ohne weiteres realisieren, wenn die Pufferlösung dann nicht mehr gedruckt werden kann. Ein weiterer Nachteil des Verfahrens besteht darin, dass die Rezeptoren in der Pufferlösung löslich sein müssen, damit sie auf den Oberflächenbereich des Trägers aufgeduckt werden können.

Aus WO 2004/050830 A ist ferner ein Verfahren zum Herstellen eines Sensorchips bekannt, bei dem ein Träger aus Glas bereitgestellt wird, der einen Oberflächenbereich hat, der eine schwach reaktive Gruppe enthält, nämlich Aminophenyl Trimethoxysilane (AMTS). Der Oberflächenbereich wird mit einer starken Säure behandelt, nämlich HCL, wobei das AMTS durch eine chemische Reaktion in Diazonium-Salz umgewandelt wird. Danach wird der Träger zum Entfernen von noch vorhandenen Säureresten und Reaktionsbestandteilen mit einer Pufferlösung gewaschen, die Sodium-Acetat, Ethanol und destilliertes Wasser enthält. Nach Entfernen der Waschlösung werden die Rezeptoren auf den Träger aufgebracht und kovalent an die Trägeroberfläche gebunden.

Werden die Rezeptoren in einer Lösung gelöst mit einem Nadeldrucker auf die Oberfläche aufgebracht, besteht auch hier das Problem, dass bei einer Lösung, die aufgrund der notwendiger Pufferkapazität eine relativ hohe Salzkonzentration aufweist, die Kapillare der Drucknadel leicht verstopfen kann, wenn sich in der Lösung Salzkristalle bilden. Das Problem kann zwar durch Ändern der Salzkonzentration beseitigt werden, jedoch binden dann die Rezeptoren nicht mehr so gut an die Trägeroberfläche.

Aus Ye ZHOU et al.: "Structural characterization of microcontact printed arrays of hexa(ethylene glycol)-terminated alkanethiols on gold", Langumir: The ACS Journal of Surface and Colloids, 20. Juli 2004, Bd. 20, Nr. 15, Seite 6206-6215 ist ferner ein gattungsfremdes Verfahren zum Herstellen eines Sensorchips bekannt, der an seiner Oberfläche mehrere voneinander beabstandete Bereiche aufweist, in denen mittels eines Stempels oberflächenaktive Rezeptormoleküle aufgebracht werden. Dazu wird der Stempel zunächst mit einer die Rezeptormoleküle enthaltenden Lösung in Kontakt gebracht, dann wird die Lösung getrocknet und danach wird der Stempel derart auf einem Gold-Substrat positioniert, dass die Rezeptormoleküle auf das Substrat übertragen werden. Dann werden Protein-Liganden mit Hilfe eines Piezo-Dispensers auf die Bereiche aufgetragen.

Aus DE 44 36 173 C1 ist ein gattungsfremdes Verfahren bekannt, bei dem eine Matrixoberfäche, wie zB. die Oberfläche eines Gels, mit photochemischen Crosslinkern modifiziert wird. An die so erhaltene Matrixoberfläche werden in Gegenwart eines Salzes zu untersuchende Moleküle gebunden und immobilisiert. Werden die Moleküle in einer Lösung gelöst mit einem Nadeldrucker auf die Oberfläche aufgebracht, kann die Kapillare der Drucknadel leicht verstopfen kann, wenn sich in der Lösung Salzkristalle bilden.

Bei einem aus Yadavalli et al., Sensors and Actuators 2004, Seite 290-297 bekannten Verfahren wird eine Silizium-Oberfläche mit einem Silan modifiziert. Auf die so erhaltene Oberfläche wird eine Photomaske aufgebracht und mit UV-Licht bestrahlt, um Polymerspots zu erzeugen. Das so erhaltene Array wird über Nacht in PBS inkubiert. Das Array weist also eine Oberflächenbeschichtung auf, die einen Rezeptor und eine Puffersubstanz enthält.

Es besteht deshalb die Aufgabe, ein Verfahren der eingangs genannten Art zu schaffen, das es eine effektive kovalente Bindung des Rezeptors an den Oberflächenbereich des Trägers ermöglicht, bei welchem Verfahren die Rezeptoren aber dennoch auf einfache Weise auf den Oberflächenbereich des Trägers aufgebracht werden können, beispielsweise mit Hilfe eines eine Kapillare aufweisenden Druckers. Ferner besteht die Aufgabe, einen Sensorchip-Rohling der eingangs genannten Art zu schaffen, der für eine Verwendung bei dem Verfahren geeignet ist.

Diese Aufgabe wird bezüglich des Verfahrens mit den Merkmalen des Anspruchs 1 gelöst.

Es wird also zunächst die Pufferlösung ohne den Rezeptor vorzugsweise in Form von matrixförmig in mehreren Reihen und/oder Spalten angeordneten Pufferlösungsvolumina auf den Träger aufgebracht und erst danach wird der Rezeptor mit der durch das Aufbringen der Pufferlösung erhaltenen Oberflächenbeschichtung des Trägers in Kontakt gebracht. Der Rezeptor wird dabei bevorzugt in Lösung auf die Oberflächenbeschichtung aufgebracht, wobei sich das Lösungsmittel, in dem der Rezeptor gelöst ist, von der Pufferlösung unterscheidenden kann. In vorteilhafter Weise ist es dadurch möglich, ein Lösungsmittel zu wählen, das ein einfaches Auftragen des Rezeptors ermöglicht, ohne dass die Gefahr besteht, dass vor oder während des Aufbringens des Rezeptors Feststoffe aus der Lösung auskristallisiert, ausgefällt und/oder ausgeschieden werden. Dennoch kann in dem Oberflächenbereich des Trägers eine hohe Konzentration der Pufferlösung bzw. einer darin enthaltenen Puffersubstanz erreicht werden, so dass der Rezeptor schnell und effektiv kovalent an die wenigstens eine, in dem Oberflächenbereich befindliche reaktive Gruppe des Trägers kovalent gebunden werden kann. Die kovalente Bindung kann über radikalische Prozesse und/oder nukleophile Additionen erfolgen. Auf dem Träger können an mehreren Stellen gleichzeitig und/oder zeitlich versetzt zueinander Untersuchungen vorgenommen werden.

Bei einer vorteilhaften Ausführungsform der Erfindung wird nach dem Aufbringen der Pufferlösung ein in dieser enthaltenes Lösungsmittel vorzugsweise durch Verdunsten derart von dem Oberflächenbereich entfernt, dass eine in der Pufferlösung enthaltene Puffersubstanz als fester, pastöser und/oder gelartiger Beschichtungsbereich auf dem Träger zurückbleibt, und dass danach auf diesen Beschichtungsbereich der Rezeptor aufgebracht wird. Der durch das aufbringen der Pufferlösung erhaltene Sensorchip-Rohling, auf dem noch keine Rezeptoren immobilisiert sind, kann dann auf einfache Weise gehandhabt werden und lässt sich außerdem gut zwischenlagern. Durch das Entfernen des Lösungsmittels lassen sich besonders hohe Puffersubstanzkonzentrationen in dem Oberflächenbereich erzielen, so dass dann der Rezeptor mit entsprechend. hoher Konzentration auf den Träger aufgebracht und auf diesem immobilisiert werden kann.

Vorteilhaft ist, wenn die Pufferlösung mit Hilfe eines die Pufferlösung mit Hilfe eines Druckverfahrens, insbesondere Tampondruckverfahrens auf den mindestens einen Oberflächenbereich des Trägers aufgebracht wird. Dabei ist es sogar möglich, mehrere oder alle Pufferlösungsvolumina der Matrix gleichzeitig auf den Träger aufzudrucken. Das erfindungsgemäße Verfahren lässt sich dadurch schnell und kostengünstig anwenden. Die Pufferlösung kann bei einer höheren Temperatur auf den mindestens einen Oberflächenbereich des Trägers aufgebracht werden als der mindestens eine Rezeptor. Dadurch wird bei wärmeempfindlichen Rezeptoren eine längere Haltbarkeitsdauer ermöglicht. Außerdem wird die Gefahr, dass die Pufferlösung kristallisiert, bevor sie auf den Träger gelangt, reduziert.

Besonders vorteilhaft ist, wenn der mindestens eine Rezeptor in einem Lösungsmittel gelöst und danach mit Hilfe eines Strahldruckers auf den Oberflächenbereich aufgedruckt oder aufgesprüht wird. Das Verfahren lässt sich dadurch noch kostengünstiger anwenden. Damit der Rezeptor genau auf der zuvor durch das Auftragen der Pufferlösung auf dem Träger erzeugten Oberflächenbeschichtung positioniert werden kann, kann ein optischer Sensor zum Erkennen der Lage der Oberflächenbeschichtung relativ zu einer Austrittsöffnung oder Düse des Strahldruckers vorgesehen sein. Der Sensor kann mit einer Positioniereinrichtung verbunden sein, welche die Austrittsöffnung in Abhängigkeit von einem Sensor-Messsignal so positioniert, dass der Rezeptor direkt auf der Oberflächenbeschichtung abgesetzt wird.

Bezüglich des eingangs erwähnten Sensorchip-Rohlings wird die vorstehend genannte Aufgabe mit den Merkmalen des Anspruchs 5 gelöst.

Die Beschichtungsbereiche sind vorzugsweise matrixformig in mehreren Reihen und/oder Spalten angeordnet. Aus dem Rohling, der an seiner Oberfläche zunächst nur die Puffersubstanz ohne den Rezeptor aufweist, kann auf einfache Weise ein Sensorchip bzw. ein Micro-Array hergestellt werden, indem ein in einem Lösungsmittel gelöster, für einen nachzuweisenden Liganden bindungsspezifischer Rezeptor auf die Puffersubstanz aufgebracht wird, um den Rezeptor auf der Oberfläche des Rohlings zu immobilisieren. Dabei bewirkt die Puffersubstanz, dass der pH-Wert der auf den Rohling aufgetragenen Lösung in einem vorbestimmten

Bereich liegt, der so gewählt ist, dass der Rezeptor zuverlässig und mit großer Effektivität kovalent an die reaktive Gruppe des Trägers bindet. Die auf den Sensorchip-Rohling aufzubringende Rezeptor-Lösung braucht deshalb selbst keine Puffersubstanz zu enthalten, wodurch die Gefahr, dass Feststoffe aus der Lösung auskristallisiert, ausgefällt und/oder ausgeschieden werden, vermieden wird. Der erfindungsgemäße Sensorchip-Rohling kann auf einfache Weise mit Hilfe eines Nadeldruckers und/oder Piezostackdruckers mit dem Rezeptor bedruckt werden, ohne das die Gefahr eines Verstopfens der Düse des Druckers durch Bildung von Kristallen besteht. Dabei kann das Aufbringen des mindestens einen Rezeptors vom Anwender des Sensorchips selbst vorgenommen werden, so dass der Anwender je nach Art und Menge der aufgebrachten Rezeptoren auf einfache Weise unterschiedliche, an die jeweils durchzuführende Messung angepasste Sensorchips herstellen kann.

Der Oberflächenbereich, in dem die wenigstens eine reaktive Gruppe des Trägers angeordnet ist, ist chemisch inert, d.h. er reagiert weder mit der Pufferlösung noch mit einem darauf aufzubringenden Rezeptor. Der Träger kann einen Grundkörper mit einer darauf angeordneten, bifunktionelle Moleküle enthaltende Oberflächenschicht aufweisen. Dabei haben die bifunktionellen Moleküle eine erste reaktive Gruppe, die an den Grundkörper bindet, und eine zweite reaktive Gruppe, die kovalent an den Rezeptor bindet. Es ist aber auch möglich, dass der Grundkörper selbst die reaktive Gruppe aufweist und somit direkt an den Rezeptor bindet.

Der Grundkörper kann aus einem Metall- oder Halbmetalloxid bestehen, wie z.B. Siliziumoxid, Aluminiumoxid, Quarzglas oder Glas oder kann eine Obefiächenschicht aus einem solchen Werkstoff aufweisen. Der Grundkörper kann auch aus einem Polymerwerkstoff bestehen oder eine Oberflöchenshicht aus einem solchen aufweisen. Der Polymerwerkstoff kann ein Cycloolefincopolymer oder ein Derivat davon, Polystyrol oder ein Derivat davon, Polyimid oder ein Derivat davon und/oder Polymetylmethacrylat oder ein Derivat davon enthalten. Der Grundkörper oder eine Oberflächenschicht des Grundkörpers kann ferner quellbare oder wasserpermeable polymere oder copolymere Strukturen aufweisen und bi- oder polyfunktionale Kopplungsgruppen umfassen. Ferner kann der Grundkörper aus einem Metall oder Halbmetall-Werkstoff bestehen oder eine Oberflächenschicht aus einem solchen Werkstoff aufweisen, wie z.B. Silizium. Der Oberflächenbereich, in dem die wenigstens eine reaktive Gruppe angeordnet ist, ist vorzugsweise eben. Er kann aber auch uneben sein, beispielsweise, wenn unter dem Oberflächenbereich Strukturen für eine elektronische Schaltung angeordnet sind, insbesondere für einen optischen Sensor zum Detektieren von Lumineszenzstrahlung und/oder eine optische Strahlungsquelle zur Aussendung einer Anregungsstrahlung.

Bei einer vorteilhaften Ausgestaltung der Erfindung sind auf dem Träger mindestens zwei Oberflächenbereiche vorgesehen sind, in denen jeweils wenigstens eine Puffersubstanz angeordnet ist, wobei sich die den einzelnen Oberflächenbereichen zugeordneten Puffersubstanzen in ihrem pH-Wert voneinander unterscheiden. Auf den Oberflächenbereichen können unterschiedliche Arten von Rezeptoren immobilisiert werden.

Vorteilhaft ist, wenn die wenigstens eine Puffersubstanz mindestens einen nicht reaktiven Farbstoff enthält. Die Puffersubstanz ist dann auf dem Träger optisch gut sichtbar und/oder mit einem optischen Sensor detektierbar. Der Rezeptor kann dadurch mit großer Präzision mit der Puffersubstanzen in Kontakt gebracht werden. Der Farbstoff kann beispielsweise Metylenblau sein.

Zweckmäßigerweise hat der Sensorchip-Rohling für die unterschiedlichen Puffersubstanzen jeweils ein maschinenlesbares Codierungselement. Das Codierungselement kann beispielsweise einen Farbcode und/oder eine elektronisches Speicherelement umfassen, in dem ein der Puffersubstanz zugeordneter Code abgelegt ist. Der Code kann dann mit einem entsprechenden Lesegerät ausgelesen werden, um die einzelnen Puffersubstanzen zu identifizieren und danach einen der betreffenden Puffersubstanz zugeordneten Rezeptor auf der Puffersubstanz aufzubringen.

Bei einer bevorzugten Ausführungsform der Erfindung ist in dem mindestens einen Beschichtungsbereich ein Indikatorfarbstoff angeordnet, dessen Farbe vom pH-Wert abhängig ist. Auf den Beschichtungsbereich kann dann ein Analyt mit einem von dem pH-Wert der in dem Beschichtungsbereich angeordneten Puffersubstanz abweichenden pH-Wert aufgebracht werden, so dass sich danach in dem Analyten ein pH-Wert einstellt, der zwischen dem pH-Wert der Puffersubstanz und demjenigen pH -Wert liegt, den der Analyt vor dem Inkontaktbringen mit der Puffersubstanz hatte. Die auf diese Weise in dem Beschichtungsbereich hervorgerufene pH-Wertänderung kann mit Hilfe des Indikatorfarbstoffs zur Anzeige gebracht werden. Dadurch lässt sich auf einfache Weise überprüfen, ob der Analyt mit dem Beschichtungsbereich in Kontakt geraten ist und somit an der vorgesehenen Stelle auf dem Träger abgesetzt wurde. Der pH-Wert des Analyten kann vor dem Inkontaktbringen des Analyten mit der Puffersubstanz ggf durch Mischen des Analyten mit einer Säure oder einer Base auf einen definierten Wert eingestellt werden.

Vorteilhaft ist, wenn auf dem Träger mehrere, seitlich voneinander beabstandete, vorzugsweise matrixförmig in mehreren Reihen und/oder Spalten angeordnete Beschichtungsbereiche vorgesehen sind. Aus dem Sensorchip-Rohling kann dann durch Aufbringen entsprechende Rezeptoren auf einfache Weise ein Micro-Array hergestellt werden.

Im Rahmen der Erfindung ist die Puffersubstanz ein wasserlösliches Salz, insbesondere Natriumphosphat. Derartige Salze sind kostengünstig verfügbar und ermöglichen hohe Pufferkapazitäten.

Vorteilhaft ist, wenn die in dem Oberflächenbereich des Trägers angeordnete reaktive Gruppe eine chemoreaktive Gruppe ist. Beispielsweise eignen sich im Falle von aminoterminierten Oligonuktleotiden sogenannte reaktive Ester oder Reaktivester, wie N-Hydroxysuccinimide (NHS-Ester), Epoxide, vorzugsweise Glycidyl-Derivate, Isothiocyanate, Iscyanate, Azide, Carbonsäuregruppen oder Maleinimide. Zur Immobilisierung der Rezeptoren können beliebige Techniken angewendet werden, beispeisweise die von G. T. Hermanson in Bioconjuncate Techniques", Academic Press, 1996, beschriebenen.

Bei einer zweckmäßigen Ausgestaltung der Erfindung ist die reaktive Gruppe eine photoreaktive Gruppe, die vorzugsweise dazu geeignet ist, bei Einwirkung von Wärme und/oder optischer Strahlung freie Radikale zu induzieren. Eine derartige Gruppe wird auch als photoreaktiver Vernetzer oder Crosslinker bezeichnet. Sie kann mindestens eine unter Benzophenon oder einem Derivat davon, Anthrachinon oder einem Derivat davon, Thymidin oder einem Derivat davon und 4-Azidobenzoesäure oder einem Derivat davon ausgewählte Gruppe umfassen.

Bei einer bevorzugten Ausgestaltung der Erfindung enthalten mehrere Beschichtungsbereiche wenigstens ein Tensid. Durch das Tensid werden Rezeptoren, die gefaltete Moleküle enthalten, nach dem Kontakt mit der Oberfläche des Sensorchip-Rohlings entfaltet. Die Moleküle können dadurch noch besser auf dem Rohling immobilisiert werden. Außerdem dient das Tensid dazu, schwer lösliche Rezeptoren in der Lösung zu halten.

Das Tensid ist bevorzugt unter Natriumpalmitat, Brij^{®} 35, Brij^{®} 58, Cetylpyridiniumchlorid-Monohydrat, Cetyltrimethylammoniumbromid, 3-(3-Cholamidopropyl)-dimethylammonio-I-propansulfonat, 3-(3-Cholamidopropyl)-dimethylammonio-2-hydroxy-l-propansulfonat, Decan-l-sulfonsäure-Natriumsalz, N,N-Bis-[3-(D-gluconamido)-propyl]-deoxycholamid, Dodecan-1-sulfonsäure-Natriumsalz, Dodecyl-β-D-maltosid, 6-0-(N-Heptylcarbamoyl)-methyl-a-D-glucopyranosid, Heptan-1-sulfonsäure-Natriumsalz, N-Lauroylsarcosin-Natriumsalz, Octanoyl-N-methylglucamid, N-Nonaoyl-N-methylglucamid, Natriumcholat, Natriumdeoxycholat, Nonan-1-sulfonsäure-Natriumsalz, Nonidet P40, Octan-1 sulfonsäure-Natriumsalz, n-Octyl-β-D-glucopyranosid, Pentan-I-sulfonsäure-Natriumsalz, n-Octyl-β-D-thioglucopyranosid, Pluronic® F -68, Saccharosemonolaurat, Natriumdodecylsulfat, N-Dodecyl-dimethyl-3-ammonio-1-propansulfonat, N-Tetradecyl-dimethyl-3-ammonio-1-propansulfonat, Triton® X-100 und/oder Mischungen davon ausgewählt. Bei diesen Tensiden konnte in der Praxis eine gute kovalente Bindung von Rezeptoren an die Oberfläche des Trägers beobachtet werden.

In der folgenden Tabelle sind weitere Details der oben genannten Tenside angegeben:

| Bezeichnung | Summenformel |
|---|---|
| Brij^{®} 35 | C₅₈H₁₁₈O₂₄ |
| Brij^{®} 58 | C₅₈H₁₁₄O₂₁ |
| Cetylpyridiniumchlorid-Monohydrat | C₂₁H₃₈ClN x H₂O |
| Cetyltrimethylamoniumbromid | C₁₉H₄₂BrN |
| (3-(3-Cholamidopropyl)-dimethylammonio-1-propansulfonat) | C₃₂H₅₈N₂O₇S |
| (3-(3-Cholamidopropyl)-dimethylammonio-2-hydroxy-1 -propansulfbnat) | C₃₂H₅₈O₇N₂O₈S |
| Dean-1-sulfonsäure-Natriumsalz | C₁₀H₂₁NaO₃S |
| (N,N-Bis-[3-(D-gluconamido]-propyl]-deoxycholamid) | C₄₂H₇₅N₃O₁₆ |
| Dodecan-1-sulfonsäure-Natriumsalz | C₁₂H₃₅NaO₃S |
| Dodecyl-β-D-maltosid | C₁₂H₃₅NaO₃S |
| (6-O-(N-Heptylcarbamoyl)-methyl-methyl-α-D-glucopyranosid) | C₁₅H₂₉NO₇ |
| Heptan-1-sulfonsäure-Natriumsalz | C₇H₁₅NaO₃S x H₂O |
| N-Lauroylsarcosin-Natriumsalz | C₁₅H₂₈NNaO₃ |
| Octanoyl-N-methylglucamid | C₁₅H₃₁NO₆ |
| N-Nonanoyl-N-methylglucamid | C₁₆H₃₃NO₆ |
| Natriumcholat | C₂₄H₃₉NaO₅ |
| Natriumdeoxycholat | C₂₄H₃₉NaO₄ |
| Nonan-1-sulfonsäure-Natriumsalz | C₉H₁₉NaO₃S |
| Nonidet P40 | Mischung aus 15 Homologen |
| Octan-1-sulfonsäure-Natriumsalz | C₈H₁₇NaO₃S |
| n-Octyl-B-D-glucopyranosid | C₁₄H₂₈O₆ |
| Pentan-1-sulfonsäure-Natriumsalz | C₅H₁₁NaO₃S |
| n-Octyl-β-D-thioglucopyranosid | C₁₄H₂₈O₅S |
| Pluronic^{®} F-68 | |
| Saccharosemonolaurat | C₂₄H₄₄O₁₂ |
| Natriumdodecylsulfat | C₂₄H₄₄O₁₂ |
| N-Dodecyl-dimethyl-3-ammonio-1-propansulfat | C₁₇H₃₇NO₃S |

| | |
|---|---|
| N-Tetradecyl-dimethyl-3-ammonio-1-propansulfat | C₁₉H₄₁NO₃S |
| Triton^{®} X-100 | |
| Triton^{®} X-1 14 | |
| Tween^{®} 20 | |
| Tween^{®} 80 | |

Ein weiterer Aspekt der Erfindung betrifft einen Kit zur Verwendung in dem erfindungsgemäßen Verfahren, der den erfindungsgemäßen Sensorchip-Rohling und zumindest eine Rezeptorlösung aufweist, die den mindestens einen Rezeptor in einem Lösungsmittel gelöst enthält. Mit Hilfe dieses Kits lassen sich durch Aufbringen der Rezeptorlösung auf den die Puffersubstanz enthaltenden Oberflächenbereich des Sensorchip-Rohlings auf einfache Weise Sensorchips herstellen. Dabei kann die Rezeptorlösung gegebenenfalls vor dem Auftragen verdünnt werden. Da die Puffersubstanz erst beim Auftragen der Rezeptorlösung auf den Sensorchip-Rohling mit der Rezeptorlösung in Kontakt gerät, lässt sich der Kit über eine längere Zeitdauer stabil lagern.

Nachstehend ist ein Ausführungsbeispiel der Erfindung anhand der Zeichnung näher erläutert. Es zeigen:
- Fig. 1: eine Seiteneinsicht eines Trägers für einen Sensorchip-Rohling,
- Fig. 2: eine Seiteneinsicht eines Sensorchip-Rohlings und
- Fig. 3: eine Seiteneinsicht eines Sensorchips.

Bei einem Verfahren zum Herstellen eines biologischen Sensorchips 1 wird ein Träger 2 bereitgestellt, der einen Oberflächenbereich 3 aufweist, in dem in der Zeichnung nicht näher dargestellte reaktive Gruppen angeordnet sind. Die reaktiven Gruppen können beispielsweise Amino- oder Epoxygruppen sein.

Der Träger 2 weist bevorzugt einen festen Grundkörper auf, auf dem eine dünne Oberflächenschicht vorgesehen ist, welche die reaktive Gruppen enthält. Die Oberflächenschicht ist mit dem Träger 2 verbunden und haftet an diesem an.

Das Auftragen der reaktiven Gruppen kann mittels DIP Coating erfolgen, indem der Grundkörper in eine Lösung eingetaucht wird, die bifunktionelle Moleküle (sogenannte "Linker") enthält, welche jeweils die bereits erwähnte reaktive Gruppe und eine Gruppe zur Kopplung an die Oberfläche des Grundkörpers aufweisen. Die zuletzt genannte Gruppe kann eine Halogensilan- (z..B. Chlorsilan) oder Alkoxysilangruppe sein.

In einem weiteren Schritt wird der Grundkörper mit einer Geschwindigkeit aus der Lösung herausgezogen, die derart gewählt ist, dass die Moleküle nach dem Herausziehen als selbstorganisierender Monolayer (SAM) auf dem Grundkörper verbleiben. Die so erhaltene Oberflächenschicht wird durch Vernetzen kovalent auf dem Grundkörper fixiert.

Es ist aber auch denkbar, dass der Werkstoff des Grundkörpers selbst die reaktiven Gruppen enthält. In diesem Fall kann das Beschichten des Grundkörpers mit der Oberflächenschicht entfallen.

Der Grundkörper besteht aus einem Werkstoff, der gegen Rezeptoren, die in einem weiteren, nachstehend noch näher zu erläuternden Verfahrensschritt auf den Träger 2 aufgebracht werden, inert ist. Der Grundkörper kann beispielsweise ein Glasplättchen, ein Polymerplättchen oder ein Halbleiterchip sein.

Der Träger 2 wird nun mit einer Pufferlösung beschichtet, die aus einem Lösungsmittel und eineidarin gelöstenPuffersubstanz, die ein Salz ist, besteht. Die Pufferlösung kann beispielsweise eine phosphatgepufferte Salzlösung (PBS-Lösung, 10-50 mM), ein Natriumphosphatpuffer (100-500 mM), ein Natrium-Citrat-Chlorid-Puffer (SSC-Puffer, 1 x-5x) oder eine Betain-Lösung sein. In der Pufferlösung kann ferner ein Tensid in einer Konzentration von 0,001-1 % enthalten sein, beispielsweise Triton^{®} X-100 und/oder Tween^{®} 20.

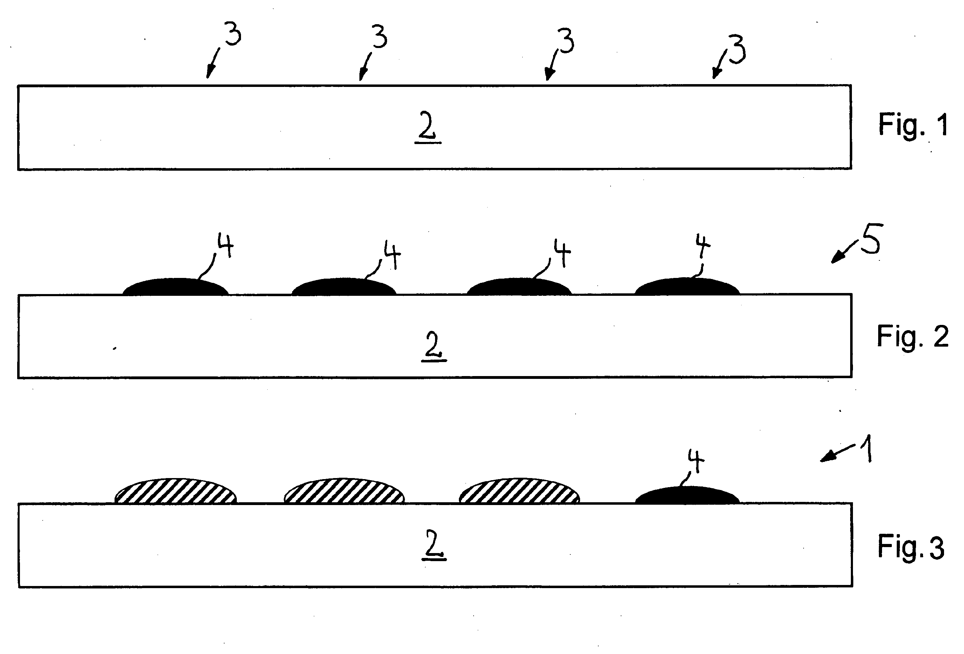

In Fig. 2 ist erkennbar, dass der Träger 2 an mehreren matrixförmig an seiner Oberfläche angeordneten, voneinander beabstandeten Stellen jeweils mit der Pufferlösung beschichtet wird. Das Aufbringen der Pufferlösung erfolgt bevorzugt mit Hilfe eines Tampondruckverfahrens, wobei mehrere zu beschichtende Stellen des Trägers 2 gleichzeitig beschichtet werden können. Die Pufferlösung kann aber auch mittels eines Nadeldruckers, eines Piezostackdruckers oder auf andere Weise auf den Träger 2 aufgebracht werden.

Das in der auf dem Träger 2 befindlichen Lösung enthaltene Lösungsmittel wird anschließend verdunstet, so dass dann an den einzelnen beschichteten Stellen jeweils die Puffersubstanz und ggf das Tensid jeweils als fester, pastöser und/oder gelartiger Beschichtungsbereich 4 auf dem Träger 2 verbleibt (verbleiben). Der so erhaltene Sensorchip-Rohling 5 kann bedarfsweise zwischengelagert werden, wobei der Zwischenlagerung in trockener Umgebung erfolgen sollte, damit der Beschichtungsbereich 4 keine Feuchtigkeit aufnimmt.

In einem weiteren Verfahrensschritt wird eine die Rezeptoren enthaltende Lösung auf die Beschichtungsbereiche 4 aufgebracht, vorzugsweise mit Hilfe eines Nadel- oder Piezostackdruckers. Die Rezeptoren sind bindungsspezifisch für einen bestimmten Liganden. Nachdem diese Lösung mit den Beschichtungsbereichen 4 in Kontakt geraten ist, binden die in der Lösung enthaltenen Rezeptoren kovalent an die an der Oberfläche des Sensorchip-Rohlings 5 befindlichen reaktiven Gruppen. Danach wird die Lösung mit eventuellen, darin noch verbliebenen freien Rezeptoren von dem Sensorchip-Rohling 5 entfernt, beispielsweise durch Waschen.

Mit dem so erhaltenen Sensorchip 1 können Liganden in einem zu untersuchenden Analyten nachgewiesen werden. Der Analyt wird dazu in flüssiger Form auf die Beschichtungsbereiche 4 aufgebracht, damit eventuelle, in dem Analyten enthaltene Liganden an die auf dem Träger 2 immobilisierten Rezeptoren binden können. Danach wird der Analyt derart von dem Sensorchip 1 abgewaschen, dass nur noch die gebundenen Liganden auf dem Sensorchip 1 verbleiben. In Abhängigkeit von der Bindung des Liganden an den Rezeptor wird dann in an sich bekannter Weise eine Lumineszenzstrahlung erzeugt und mit Hilfe eines optischen Sensors detektiert. Bei dem in Fig. 3 gezeigten Ausführungsbeispiel wurden auf einer der Beschichtungsbereiche 4 des Sensorchip-Rohlings 5 keine Rezeptoren immobilisiert. Dieser Beschichtungsbereich 4 kann für Referenz- oder Blindmessungen verwendet werden.

## Patentansprüche

1. Verfahren zum Herstellen eines Sensorchips (1), wobei ein Träger (2) bereitgestellt wird, der mindestens einen, wenigstens eine reaktive Gruppe enthaltenden Oberflächenbereich (3) hat, und wobei der Oberflächenbereich (3) durch Aufbringen einer Pufferlösung mit einer Oberflächenbeschichtung versehen wird, **dadurch gekennzeichnet, dass** die Pufferlösungderart auf den Oberflächenbereich (3) aufgebracht wird, dass auf dem Träger (2) mehrere, seitlich voneinander beabstandete Pufferlösungsvolumina gebildet sind, und dass danach mit der Oberflächenbeschichtung mindestens ein für einen nachzuweisenden Liganden bindungsspezifischer Rezeptor derart in Kontakt gebracht wird, dass er an die mindestens eine reaktive Gruppe kovalent bindet.

2. Verfahren nach Anspruch 1, **dadurch gekennzeichnet, dass** nach dem Aufbringen der Pufferlösung ein in dieser enthaltenes Lösungsmittel vorzugsweise durch Verdunsten derart von dem Oberflächenbereich (3) entfernt wird, dass eine in der Pufferlösung enthaltene Puffersubstanz als fester, pastöser und/oder gelartiger Beschichtungsbereich (4) auf dem Träger (2) zurückbleibt, und dass danach auf diesen Beschichtungsbereich (4) der Rezeptor aufgebracht wird.

3. Verfahren nach Anspruch 1 oder 2, **dadurch gekennzeichnet, dass** die Pufferlösung mit Hilfe eines Druckverfahrens, insbesondere eines Tampondruckverfahrens auf den mindestens einen Oberflächenbereich (3) des Trägers (2) aufgebracht wird.

4. Verfahren nach einem der Ansprüche 1 bis 3, **dadurch gekennzeichnet, dass** der mindestens eine Rezeptor in einem Lösungsmittel gelöst und danach mit Hilfe eines Strahldruckers auf den Oberflächenbereich (3) aufgedruckt oder aufgesprüht wird.

5. Sensorchip-Rohling (5), der einen Träger (2) hat, der mindestens einen Oberflächenbereich (3) aufweist, in dem wenigstens eine reaktive Gruppe enthalten ist, die dazu geeignet ist, kovalent an einen Rezeptor zu binden,
**dadurch gekennzeichnet, dass** auf dem Oberflächenbereich (3) mehrere, seitlich voneinander beabstandete feste, pastöse und/oder gelartige Beschichtungsbereiche (4) angeordnet sind, die aus einer Puffersubstanz bestehen, die ein Salz ist.

6. Sensorchip-Rohling (5) nach Anspruch 5, **dadurch gekennzeichnet, dass** sich die Puffersubstanzen von mindestens zwei Oberflächenbereichen (3) in ihrem pH-Wert voneinander unterscheiden.

7. Sensorchip-Rohling (5) nach Anspruch 5 oder 6, **dadurch gekennzeichnet, dass** die wenigstens eine Puffersubstanz mindestens einen nicht reaktiven Farbstoff enthält.

8. Sensorchip-Rohling (5) nach einem der Ansprüche 5 bis 7, **dadurch gekennzeichnet, dass** er für die unterschiedlichen Puffersubstanzen jeweils ein maschinenlesbares Codierungselement aufweist.

9. Sensorchip-Rohling (5) nach einem der Ansprüche 5 bis 8, **dadurch gekennzeichnet, dass** in dem mindestens einen Beschichtungsbereich (4) ein lndikatorfarbstoff angeordnet ist, dessen Farbe vom pH-Wert abhängig ist.

10. Sensorchip-Rohling (5) nach einem der Ansprüche 5 bis 9, **dadurch gekennzeichnet, dass** die Puffersubstanz ein wasserlösliches Salz ist, insbesondere Natriumphosphat.

11. Sensorchip-Rohling (5) nach einem der Ansprüche 5 bis 10, **dadurch gekennzeichnet, dass** die reaktive Gruppe eine chemoreaktive Gruppe ist.

12. Sensorchip-Rohling (5) nach einem der Ansprüche 5 bis 11, **dadurch gekennzeichnet, dass** die reaktive Gruppe eine photoreaktive Gruppe ist, die vorzugsweise dazu geeignet ist, bei Einwirkung von Wärme und/oder optischer Strahlung freie Radikale zu induzieren.

13. Sensorchip-Rohling (5) nach einem der Ansprüche 5 bis 12, **dadurch gekennzeichnet, dass** mehrere Beschichtungsbereiche (4) wenigstens ein Tensid enthalten.

14. Sensorchip-Rohling (5) nach einem der Ansprüche 5 bis 1 3, **dadurch gekennzeichnet, dass** das Tensid unter Natriumpalmitat, Brij^{®}35, Brij^{®}58, Cetylpyridiniumchlorid-Monohydrat, Cetyltrimethylammoniumbromid, 3-(3-Cholamidopropyl)-dimethylammonio-I-propansulfonat, 3-(3-Cholamidopropyl)-dimethylammonio-2-hydroxy-I-propansulfonat, Decan-1-sulfonsäure-Natriumsalz, N,N-Bis-[3-(D-gluconamido)-propyl]-deoxycholamid, Dodecan-1-sulfonsäure-Natriumsalz, Dodecyl-β-D-maltosid, 6-0-(N-Heptylcarbamoyl)-methyl-a-D-glucopyranosid, Heptan-1-sulfonsäure-Natriumsalz, N-Lauroylsarcosin-Natriumsalz, Octanoyl-N-methylglucamid, N-Nonaoyl-N-methylglucamid, Natriumcholat, Natriumdeoxycholat, Nonan-1-sulfonsäure-Natriumsalz, Nonidet P40, Octan-1 sulfonsäure-Natriumsalz, n-Octyl-β-D-glucopyranosid, Pentan-I-sulfonsäure-Natriumsalz, n-Octyl-β-D-thioglucopyranosid, Pluronic^{®} F-68, Saccharosemonolaurat, Natriumdodecylsulfat, N-Dodecyl-dimethyl-3-ammonio-1-propansulfonat, N-Tetradecyl-dimethyl-3-ammonio-1-propansulfonat, Triton^{®} X-100 und/oder Mischungen davon ausgewählt ist.

15. Kit zur Verwendung in einem Verfahren nach einem der Ansprüche 1 bis 4, **dadurch gekennzeichnet, dass** er einen Sensorchip-Rohling (5) nach einem der Ansprüche 5 bis 14 und zumindest eine Rezeptorlösung aufweist, die den mindestens einen Rezeptor in einem Lösungsmittel gelöst enthält.

## Claims

1. Process for manufacturing a sensor chip (1), wherein a support (2) is prepared that has at least one surface region (3) comprising at least one reactive group, and wherein a buffer solution is applied to the surface region (3) in order to provide the latter with a surface coating, **characterized in that** the buffer solution is applied to the surface region (3) such that a plurality of buffer solution volumes separated laterally from one another are formed on the support (2), and **in that** thereafter at least one receptor with binding specificity for a ligand that is to be detected is brought into contact with the surface coating, such that the receptor binds covalently to the at least one reactive group.

2. Process according to Claim 1, **characterized in that**, after application of the buffer solution, a solvent contained in the buffer solution is removed from the surface region (3) preferably by evaporation, so that a buffer substance contained in the buffer solution remains on the support (2) as a solid, paste-like and/or gel-like coating region (4) and so that the receptor is then applied to said coating region (4).

3. Process according to Claim 1 or 2, **characterized in that** the buffer solution is applied by means of a printing process, in particular a pad printing process, to the at least one surface region (3) of the support (2).

4. Process according to one of Claims 1 to 3, **characterized in that** the at least one receptor is dissolved in a solvent and then printed or sprayed onto the surface region (3) using a jet printer.

5. Sensor chip blank (5) having a support (2) that has at least one surface region (3) in which at least one reactive group is present that is suitable for covalently bonding to a receptor, **characterized in that** two or more solid, paste-like and/or gel-like coating regions (4) separated laterally from one another and consisting of a buffer substance, which is a salt, are arranged on the surface region (3).

6. Sensor chip blank (5) according to Claim 5, **characterized in that** the buffer substances of at least two surface regions (3) differ from each other in their pH.

7. Sensor chip blank (5) according to Claim 5 or 6, **characterized in that** the at least one buffer substance contains at least one non-reactive dye.

8. Sensor chip blank (5) according to one of Claims 5 to 7, **characterized in that** it has a machine-readable coding element for each of the different buffer substances.

9. Sensor chip blank (5) according to one of Claims 5 to 8, **characterized in that** an indicator dye is arranged in the at least one coating region (4) and the color of said dye is pH-dependent.

10. Sensor chip blank (5) according to one of Claims 5 to 9, **characterized in that** the buffer substance is a water-soluble salt, in particular sodium phosphate.

11. Sensor chip blank (5) according to one of Claims 5 to 10, **characterized in that** the reactive group is a chemoreactive group.

12. Sensor chip blank (5) according to one of Claims 5 to 11, **characterized in that** the reactive group is a photoreactive group that is preferably suitable for inducing free radicals upon the application of heat and/or optical radiation.

13. Sensor chip blank (5) according to one of Claims 5 to 12, **characterized in that** two or more coating regions (4) contain at least one surfactant.

14. Sensor chip blank (5) according to one of Claims 5 to 13, **characterized in that** the surfactant is chosen from the group comprising sodium palmitate, Brij^{®} 35, Brij^{®} 58, cetyl pyridinium chloride monohydrate, cetyl trimethyl ammonium bromide, 3-(3-cholamidopropyl) dimethylammonio-1-propane sulfonate, 3-(3-cholamidopropyl)-dimethylammonio-2-hydroxy-1-propane sulfonate, decane-1-sulfonic acid sodium salt, N,N-bis-[3-(D-gluconamido)-propyl]-deoxycholamide, dodecane-1-sulfonic acid sodium salt, dodecyl-β-D-maltoside, 6-0-(N-heptylcarbamoyl)-methyl-a-D-glucopyranoside, heptane-1-sulfonic acid sodium salt, N-lauroylsarcosine sodium salt, octanoyl-N-methyl glucamide, N-nonanoyl-N-methyl glucamide, sodium cholate, sodium deoxycholate, nonane-1-sulfonic acid sodium salt, Nonidet P40, octane-1 sulfonic acid sodium salt, n-octyl-β-D-glucopyranoside, pentane-1-sulfonic acid sodium salt, n-octyl-β-D-thioglucopyranoside, Pluronic^{©} F-68, saccharose monolaurate, sodium dodecyl sulfate, N-dodecyl-dimethyl-3-ammonio-1-propane sulfonate, N-tetradecyl-dimethyl-3-ammonio-1-propane sulfate, Triton^{®} X-100 and/or mixtures thereof.

15. Kit for use in a process according to one of Claims 1 to 4, **characterized in that** it has a sensor chip blank (5) according to one of Claims 5 to 14 and at least one receptor solution, which contains the at least one receptor dissolved in a solvent.

## Revendications

1. Procédé de fabrication d'une puce de détecteur (1) dans lequel on prépare un support (2) qui présente au moins une zone de surface (3) qui contient au moins un groupe réactif et dans lequel la zone de surface (3) est dotée d'un revêtement de surface par application d'une solution tampon, **caractérisé en ce que** la solution tampon est appliquée sur la zone de surface (3) de manière à former sur le support (2) plusieurs volumes de solution tampon situés à distance latérale les uns des autres et **en ce qu'**ensuite on met en contact avec le revêtement de surface au moins un récepteur qui se lie spécifiquement à un ligand à détecter, de telle sorte que ce récepteur se lie par covalence audit au moins un groupe réactif présent.

2. Procédé selon la revendication 1, **caractérisé en ce qu'**après l'application de la solution tampon, on élimine de la zone de surface (3) un solvant que contient cette solution, de préférence par évaporation, **en ce qu'**une substance tampon que contient la solution tampon reste sur le support (2) sous la forme d'une zone de revêtement (4) solide, pâteuse et/ou gélifiée et **en ce qu'**ensuite on applique le récepteur sur cette zone (4) de revêtement.

3. Procédé selon la revendication 1 ou 2, **caractérisé en ce que** la solution tampon est appliquée sur ladite au moins une zone de surface (3) du support (2) à l'aide d'un procédé d'impression, de préférence d'un procédé d'impression au tampon.

4. Procédé selon l'une quelconque des revendications 1 à 3, **caractérisé en ce que** ledit au moins un récepteur est dissous dans un solvant et est ensuite imprimé ou projeté sur la zone de surface (3) à l'aide d'une imprimante à jet.

5. Ebauche (5) de puce de détecteur qui possède un support (2) qui présente au moins une zone de surface (3) qui contient au moins un groupe réactif qui convient pour se lier par covalence à un récepteur, **caractérisée en ce que** plusieurs zones de revêtement (4) solides, pâteuses et/ou gélifiées sont disposées à distance latérale les unes des autres sur la zone de surface (3) et sont constituées d'une substance tampon qui est un sel.

6. Ebauche (5) de puce de détecteur selon la revendication 5, **caractérisée en ce que** les valeurs du pH des substances tampon d'au moins deux zones de surface (3) sont différentes.

7. Ebauche (5) de puce de détecteur selon la revendication 5 ou 6, **caractérisée en ce que** ladite au moins une substance tampon contient au moins un colorant non réactif.

8. Ebauche (5) de puce de détecteur selon l'une quelconque des revendications 5 à 7, **caractérisée en ce que** pour les différentes substances tampon, elle présente un élément codé respectif lisible par machine.

9. Ebauche (5) de puce de détecteur selon l'une quelconque des revendications 5 à 8, **caractérisée en ce qu'**un colorant d'indicateur dont la couleur dépend de la valeur du pH est disposé dans ladite au moins une zone de revêtement (4).

10. Ebauche (5) de puce de détecteur selon l'une quelconque des revendications 5 à 9, **caractérisée en ce que** la substance tampon est un sel soluble dans l'eau et en particulier du phosphate de sodium.

11. Ebauche (5) de puce de détecteur selon l'une quelconque des revendications 5 à 10, **caractérisée en ce que** le groupe réactif est un groupe chimiquement réactif.

12. Ebauche (5) de puce de détecteur selon l'une quelconque des revendications 5 à 11, **caractérisée en ce que** le groupe réactif est un groupe photoréactif qui convient de préférence pour induire la formation de radicaux libres sous l'action de la chaleur et/ou d'un rayonnement optique.

13. Ebauche (5) de puce de détecteur selon l'une quelconque des revendications 5 à 12, **caractérisée en ce que** plusieurs zones de revêtement (4) contiennent au moins un agent tensioactif.

14. Ebauche (5) de puce de détecteur selon l'une quelconque des revendications 5 à 13, **caractérisée en ce que** l'agent tensioactif est sélectionné parmi le palmitate de sodium, le Brij^{®}35, le Brij^{®}58, le chlorure de cétylpyridinium monohydraté, le bromure de cétyltriméthylammonium, le diméthylammonio-1-propanesulfonate de 3-(3-cholamidopropyl), le diméthylammonio-2-hydroxy-1-propanesulfonate de 3-3-cholamidopropyl), le sel de sodium de l'acide décane-1-sulfonique, le désoxycholamide de N,N-bis-[3-(D-gluconamido)-propyl], le sel de sodium de l'acide dodécane-1-sulfonique, le β-D-maltoside de dodécyle, le méthyl-a-D-glucopyranoside de 6-0-(N-heptylcarbamoyl), le sel de sodium de l'acide heptane-1-sulfonique, le sel de sodium de N-lauroylsarcosine, le N-méthylglucamide d'octanoyle, le N-méthylglucamide de N-nonaoyle, le cholate de sodium, le désoxycholate de sodium, le sel de sodium de l'acide nonane-1-sulfonique, le Nonidet P40, le sel de sodium de l'acide octane-1-sulfonique, le β-D-glucopyranoside de n-octyle, le sel de sodium de l'acide pentane-1-sulfonique, le β-D-thioglucopyranoside de n-octyle, le Pluronic® F-68, le monolaurate de saccharose, le dodécylsulfate de sodium, le 3-ammonio-1-propanesulfonate de N-dodécyldiméthyle, le 3-ammonio-1-propanesulfonate de N-tétradécyldiméthyle, le Triton® X-100 et/ou leurs mélanges.

15. Trousse destinée à être utilisée dans un procédé selon l'une quelconque des revendications 1 à 4, **caractérisée en ce qu'**elle contient une ébauche (5) de puce de détecteur selon l'une quelconque des revendications 5 à 14 et au moins une solution de récepteur qui contient ledit au moins un récepteur dissous dans un solvant.
